(19) Europäisches Patentamt
European Patent Office
Office européen des brevets

(11) Numéro de publication: **0 187 057**
**A1**

(12) # DEMANDE DE BREVET EUROPEEN

(21) Numéro de dépôt: **85402147.4**

(22) Date de dépôt: **07.11.85**

(51) Int. Cl.⁴: **C 07 C 127/22**, A 61 K 31/27

(30) Priorité: **08.11.84 FR 8417016**

(43) Date de publication de la demande: **09.07.86**
**Bulletin 86/28**

(84) Etats contractants désignés: **CH DE FR GB IT LI NL**

(71) Demandeur: **COMPAGNIE FRANCAISE DE SUCRERIE, 336, rue Saint-Honoré, F-75001 Paris (FR)**

(72) Inventeur: **Anatol, Jésus, décédé (FR)**
Inventeur: **Foussard-Blanpin, Odette, 11 Rue du Pas d'Ane, F-37300 Joue Les Tours (FR)**

(74) Mandataire: **Rinuy, Santarelli, 14, avenue de la Grande Armée, F-75017 Paris (FR)**

(54) **Phényl-2 allophanate d'isopropyle, son procédé d'obtention et son application dans le domaine pharmaceutique.**

(57) L'invention concerne à titre de produit industriel nouveau le phényl-2 allophanate d'isopropyle de formule:

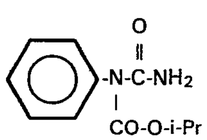

Son procédé d'obtention consiste à hydrolyser le phénylisopropyloxycarbonyle cyanamide lui-même résultant de l'acylation du phényl cyanamide par le chloroformiate d'isopropyle.

Application de ce nouvel allophanate dans le domaine pharmaceutique pour, entre autres, ses propriétés anticonvulsivantes, en association, ou non, avec du phényl-2 allophanate d'éthyle.

EP 0 187 057 A1

Phényl-2 allophanate d'isopropyle, son procédé d'obtention et son application dans le domaine pharmaceutique

La présente invention concerne, à titre de produit industriel nouveau, le phényl-2 allophanate d'isopropyle, son procédé d'obtention et son application dans le domaine pharmaceutique.

Le composé nouveau en question sera désigné, dans la suite de la présente description, et pour plus de commodité, par la désignation SCS 289.

Ce composé répond à la formule :

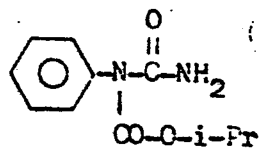

où : i-Pr représente le radical isopropyle.

De façon générale, le procédé permettant d'obtenir ce composé nouveau consiste à acyler le phényl cyanamide puis à hydrolyser le produit résultant de cette acylation.

De façon plus particulière, l'acylation est avantageusement réalisée par réaction du chloroformiate d'isopropyle sur le phényl cyanamide et de façon encore plus avantageuse, sur le dérivé métallique tel que de sodium ou de potassium de ce dernier.

La réaction d'acylation peut alors s'écrire, dans sa forme la plus générale :

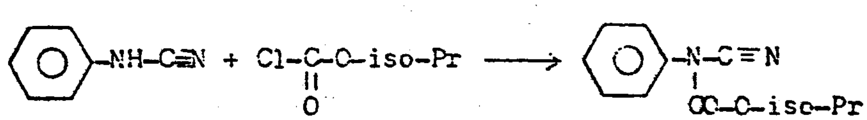

Le produit résultant de cette acylation est le phényl-isopropyloxycarbonyl cyanamide qui est un composé nouveau à la connaissance de la Demanderesse.

Le dérivé métallique (de sodium ou de potassium) du phényl cyanamide peut s'obtenir simplement en opérant dans un milieu aqueux et en dissolvant le phényl cyanamide dans une solution aqueuse contenant la

quantité équimoléculaire de soude ou de potasse. On peut également opérer en additionnant au phényl cyanamide simultanément une solution aqueuse de soude ou de potasse et le chloroformiate par une réaction de Schotten-Baumann. On peut également réaliser cette réaction par transfert de phase.

Le phénylisopropyloxycarbonyl cyanamide est ensuite hydrolysé en le composé phényl-2 allophanate d'isopropyle (SCS 289) suivant le schéma :

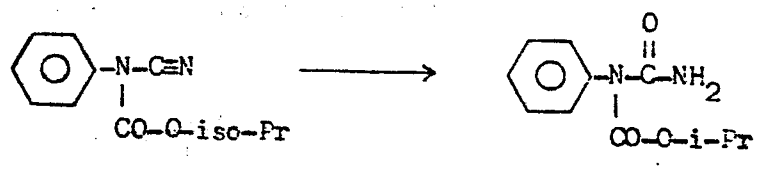

Une telle hydrolyse peut être réalisée en milieu acide par une solution d'acide chlorhydrique éventuellement en présence d'un solvant organique miscible à l'eau comme l'éthanol ou l'acide acétique pour favoriser la solubilisation du produit et en opérant à chaud par exemple à la température de reflux du mélange réactionnel. La progression de la réaction peut facilement être observée en chromatographie sur couche mince.

Les exemples suivants sont donnés à titre illustratif du procédé tel que défini ci-dessus :

<u>Exemple 1 : Phényl isopropyloxycarbonyl cyanamide (SCS 283)</u>

A 0,5 mole de phényl cyanamide dissous dans une solution aqueuse de potasse préparée à partir de 33 g de potasse en pastilles et de 200 ml d'eau et refroidie au bain de glace, on ajoute goutte à goutte, sous bonne agitation et en maintenant la température du mélange inférieure à 10°C ; 61,28 g (0,5 mole) de chloroformiate d'isopropyle.

L'addition dure environ 3 heures. Un précipité se forme rapidement. On laisse le mélange réactionnel revenir à température ambiante, puis filtre le précipité, le lave à l'eau jusqu'à neutralité et le sèche.

Poids sec obtenu : 68,25 g - Rdt : 66,8 %

F = 52 à 58°C (banc Köfler)

Ce produit brut peut être recristallisé de l'éther isopropylique :

F = 59° (banc Köfler)

Chromatographie sur couche mince de silice :

Eluant : chloroforme-méthanol (99:1) :

Rf : 0,90

Analyse $C_{11}H_{12}N_2O_2$ M = 204,223

| | C : | H : | N : |
|---|---|---|---|
| Calculé % | 64,69 | 5,92 | 13,72 |
| Trouvé % | 64,64 | 5,85 | 13,80 |

Spectre IR : Pastille KBr 1 %

Bande CN à 2250 $cm^{-1}$

Bande CO à 1755 $cm^{-1}$

Exemple 2 : Phényl-2 allophanate d'isopropyle (SCS 289)

A 75 g (0,367 mole) de phényl isopropyloxycarbonyl cyanamide, on ajoute 150 ml d'alcool absolu et 75 ml d'acide chlorhydrique concentré (d = 1,18) et chauffe au reflux pendant 2 heures.

Après refroidissement, le produit qui a précipité est essoré et lavé à l'eau jusqu'à neutralité.

Après séchage, on obtient : 52,0 g (63 %) de produit brut, F environ 100° (banc Köfler), que l'on recristallise dans 10 volumes d'alcool absolu. On obtient finalement après essorage et séchage : 36,0 g de produit pur. Rdt : 44,1 %.

F = 176°C avec sublimation (banc Köfler).

<u>Chromatographie sur couche mince de silice</u>
Eluant : chloroforme-méthanol (85:15) :
Rf = 0,65

<u>Spectre IR</u> : pastille KBr 1 %
Bandes CO à 1700 et 1720 $cm^{-1}$
Bandes NH à 3220, 3260 et 3390 $cm^{-1}$

<u>Analyse</u> : $C_{11}H_{14}N_2O_3$ M = 222,24

| | C : | H : | N : |
|---|---|---|---|
| Calculé % | 59,45 | 6,35 | 12,60 |
| Trouvé % | 59,48 | 6,40 | 12,46 |

Le produit SCS 289 selon l'invention s'est avéré être particulièrement efficace dans le domaine pharmaceutique. En effet, la Demanderesse a constaté que ce produit SCS 289 possède des propriétés anticonvulsivantes particulièrement marquées vis-à-vis des crises psychomotrices et des crises pentétrazoliques, modèles expérimentaux classiquement utilisés pour reproduire chez l'animal les crises humaines de type "troubles du comportement" ou "petit mal". Le produit exerce cette activité à des doses inférieures à celles qui dépriment le système nerveux central. Sa maniabilité est supérieure à celle du valproate de sodium.

Par ailleurs, la Demanderesse a également constaté que ce même produit SCS 289 associé à un produit appartenant à la même famille des allophanates, à savoir le phényl-2 allophanate d'éthyle (SCS 35) donne lieu à une synergie d'action à l'égard des crises pentétrazoliques.

Les tests pharmacologiques réalisés ont en effet révélé ces intéressantes propriétés. Ces dernières sont illustrées ci-après de façon succincte.

La toxicité aiguë et les propriétés anticonvulsivantes du phényl-2 allophanate d'isopropyle

ont été étudiées à l'égard de divers types de convulsions expérimentales chez le lapin et la souris ; on a de même déterminé la maniabilité du produit en utilisant l'écart entre les doses actives et celles qui dépriment le système nerveux central. Au surplus, on a constaté l'action de synergie avec le SCS 35.

Toxicité aiguë

Des doses croissantes de produit (SCS 289) ont été administrées, par voie digestive, sous forme de suspensions gommeuses, sous le volume de 0,50 ml/20 g de poids corporel chez la souris et 0,50 ml/100 g de poids corporel chez le rat (de 500 à 4000 mg/kg avec des intervalles de 500 mg/kg).

Les animaux ont été maintenus en observation pendant une semaine, tout d'abord dans le laboratoire (avec un chauffage d'appoint) jusqu'au réveil des animaux endormis, puis dans l'unité animale conditionnée et climatisée.

Le taux de mortalité relevé dans chaque lot de rats et de souris au terme de la semaine d'observation a été le suivant :

TOXICITE AIGUE DU SCS 289 PAR VOIE DIGESTIVE

| Pourcentage de létalité Souris | Doses mg/kg | Pourcentage de létalité Rats |
|---|---|---|
| 0 | 500 | 0 |
| 0 | 1000 | 15 |
| 0 | 1500 | 30 |
| 20 | 2000 | 55 |
| 40 | 2500 | 60 |
| 100 | 3000 | 65 |
| 100 | 3500 | 80 |
| 100 | 4000 | 100 |

(20 animaux par dose dans chaque groupe)

Les valeurs des $DL_{50}$, calculées par la méthode de Miller et Tainter sont respectivement de :

- 2300 ± 102 mg/kg chez la souris ;
- 2050 ± 220 mg/kg chez le rat.

Propriétés anticonvulsivantes :

On a effectué, pour déterminer ces propriétés anticonvulsivantes, des expérimentations sur le SCS 289 en procédant à des essais de protection :

- chez le lapin soumis à une transélectrification cranienne génératrice de crises épileptiformes de type "grand mal" ou recevant une infusion veineuse de pentétrazol provoquant l'apparition de myoclonies palpébrales ;
- chez la souris recevant, par voie sous-cutanée des doses croissantes de pentétrazol entrainant convulsions et mort ou soumise à une transélectrification transcranienne à basse fréquence génératrice de crises psychomotrices.

Les résultats quant à la protection contre les crises pentétrazoliques liminaires génératrices de myoclonies palpébrales figurent dans les tableaux ci-après.

INFUSIONS VEINEUSES PENTETRAZOLIQUES GENERATRICES DE MYOCLONIES PALPEBRALES CHEZ LE LAPIN

TEMOINS

| Poids des animaux en kg | Temps d'infusion veineuse° (minutes et secondes) | Quantité de pentétrazol° mg/kg |
|---|---|---|
| 3,45 | 18 | 20,87 |
| 3,83 | 22 | 22,98 |
| 2,75 | 23,30 | 34,18 |
| 2,90 | 20 | 27,58 |
| 2,50 | 15 | 24 |
| 2,80 | 15,30 | 22,14 |
| 2,48 | 15,30 | 25 |
| 2,90 | 14,30 | 20 |
| 3,75 | 17 | 18,13 |
| 3,75 | 15 | 16 |

° nécessaire à l'apparition des myoclonies palpébrales

LAPINS AYANT RECU 50 mg/kg de SCS 289

| | | |
|---|---|---|
| 2,55 | 26 | 40,78 |
| 2,63- | 28 | 42,58 |
| 2,80 | 36 | 51,43 |
| 2,55 | 21,15 | 33,33 |
| 2,90 | 31,40 | 43,68 |
| 2,75 | 22,20 | 32,44 |
| 2,70 | 15 | 22,22 |
| 2,80 | 28 | 40 |
| 2,54 | 28,40 | 45,15 |
| 3,20 | 16 | 20 |

| INFUSIONS VEINEUSES PENTETRAZOLIQUES GENERATRICES DE MYOCLONIES PALPEBRALES CHEZ LE LAPIN | | |
|---|---|---|
| LAPINS AYANT RECU 100 mg/kg de SCS 289 | | |
| Poids des animaux en kg | Temps d'infusion veineuse ° (minutes et secondes) | Quantité de pentétrazol° (mg/kg) |
| | nécessaire à l'apparition des myoclonies palpébrales | |
| 2,63 | > 45 | > 68,44 |
| 2,54 | 28,40 | 45,15 |
| 2,40 | 34 | 56,67 |
| 3,95 | 33 | 33,84 |
| 3,50 | 33 | 37,71 |
| 3,90 | 35,30 | 36,41 |
| 2,70 | 35 | 51,85 |
| 2,70 | 37 | 51,81 |
| 2,42 | 28 | 46,28 |
| 2 | 32 | 64 |

L'administration préalable de SCS 289 retarde l'apparition des myoclonies palpébrales provoquées par l'infusion veineuse de pentétrazol.

| Doses de SCS 289 (en mg/kg) | Dose moyenne de pentétrazol (mg/kg) | Elévation du seuil myoclonique |
|---|---|---|
| / | 23,0 | - |
| 50 | 37,2 | 61,7 % |
| 100 | 49,2 | 113 |

Une expérimentation similaire pratiquée en essais croisés a donné les résultats suivants pour divers anticonvulsivants de référence :

- Phénacétamide (Epiclase®) 25 mg/kg... 32,4
- " 50 mg/kg... 43,1
- " 100 mg/kg... 58,2
- Valproate de sodium (Dépakine®) 100 mg/kg... 54,7
  500 mg/kg... 65,5

Quant à l'essai de protection contre les crises électriques maximales génératrices de crises épileptiformes de type "grand mal" (1er et 2ème électrochocs) effectué chez le lapin, on constate :

- qu'à la dose de 100 mg/kg de SCS 289, il n'est jamais observé de protection complète, mais six animaux sur dix sont protégés contre la forme tonique des convulsions : protection de 60 % des animaux contre l'hyperextension tonique des pattes postérieures ;
- qu'à la dose de 200 mg/kg de SCS 289, deux animaux qui avaient fait une crise typique avant traitement, sont entièrement protégés lorsque le second électrochoc est appliqué une heure après traitement. Cinq autres lapins sont protégés contre les seules crises toniques : protection totale de 20 % des animaux, protection de cinq animaux sur dix contre la forme tonique des convulsions.

On notera que dans les mêmes conditions expérimentales, le valproate de sodium est absolument inefficace à la dose de 500 mg/kg.

Chez le lapin, le SCS 289 exerce, comme chez la souris, des effets anticonvulsivants ; son action est manifeste à l'égard du pentétrazol dès la dose de 50mg/kg qui exerce une activité quantitativement du même ordre de grandeur que le valproate de sodium à 500mg/kg. Les effets sont nettement moindres vis-à-vis de l'électrochoc générateur de crises épileptiformes dont seul l'aspect tonique est supprimé chez 60 % des lapins traités par 100 mg/kg alors que la dose double protège complètement 20 % seulement des animaux. A noter que, dans cette épreuve, le valproate de sodium est inactif à la dose de 500 mg/kg.

On a donc ainsi vérifié, chez le lapin, que le SCS 289 exerce des effets anticonvulsivants et que son action s'exerce préférentiellement à l'égard des crises pentétrazoliques.

On a également recherché si le SCS 289 était susceptible de protéger la souris contre le choc psychomoteur induit par une stimulation transcranienne à basse fréquence qui provoque, chez l'animal, un bref comportement de désorientation rappelant les attaques psychomotrices de l'homme.

Les résultats des essais effectués figurent au tableau ci-après :

| Doses de SCS 289 (mg/kg) | Pourcentage de souris protégées une heure après traitement* (Groupes de 20 souris) |
| --- | --- |
| 2,5 | 35 |
| 5 | 45 |
| 10 | 50 |
| 25 | 95 |
| 50 | 100 |

(* N'avaient été retenus pour l'essai que les animaux présentant, avant traitement, les modifications typiques de leur comportement pendant 15 secondes au moins).

L'administration de SCS 289 protège la souris contre les crises psychomotrices. La $DE_{50}$ capable de protéger 50 % des animaux est égale à 4,5 mg/kg.

Il a été procédé, dans les mêmes conditions, à une recherche de l'activité de la phénacétamide et du valproate de sodium.

En ce qui concerne la phénacétamide, la protection est incontestable, la $DE_{50}$ étant de l'ordre de 14 mg/kg.

- 10 mg/kg protègent 40 % des animaux ;
- 25 mg/kg protègent 70 % des animaux ;
- 50 mg/kg protègent 85 % des animaux ;
- 100 mg/kg protègent 100 % des animaux.

Le valproate de sodium est également susceptible de protéger la souris contre les crises psychomotrices expérimentales et ce, d'autant plus, que les doses mises en oeuvre sont plus élevées :

- 50 mg/kg protègent 15 % des animaux ;
- 100 mg/kg protègent 40 % des animaux ;
- 250 mg/kg protègent 85 % des animaux,

de sorte que la $DE_{50}$ capable de protéger 50 % des animaux est de l'ordre de 120 mg/kg.

Dans cette expérimentation, où chaque dose de produit est administrée par voie digestive à un groupe de 20 souris, la comparaison des $DE_{50}$ semble indiquer que le SCS 289 est pratiquement trois fois plus actif que la phénacétamide et 26 fois plus actif que le valproate de sodium.

En ce qui concerne la maniabilité, la comparaison des doses de SCS 289 exerçant, chez la souris, des effets létaux et anticonvulsivants permet de prévoir une marge thérapeutique assez confortable (dans le domaine du "petit mal") :

- $DL_{50}$ = 2300 ± 102 mg/kg ;
- $DE_{50}$ narcotique, comprise entre 1 et 2 g/kg;
- $DE_{50}$ anticonvulsivante vis-à-vis du pentétrazol ;
- $DE_{50}$ anticonvulsivante vis-à-vis des crises psychomotrices.

Toutefois, la maniabilité d'emploi du produit en comparant également les doses anticonvulsivantes et celles qui sont susceptibles, en déprimant le système nerveux central, de modifier le comportement psychomoteur, de façon à envisager la possibilité d'emploi en traitement ambulatoire sans entrave à la vigilance du sujet traité, a été précisée.

On a donc étudié les effets dépresseurs que peuvent exercer des doses croissantes de SCS 289 sur :

- la motricité spontanée de la souris ;
- sa curiosité, quantifiée par la méthode de la planche à trous ;
- son agilité, évaluée par l'épreuve de la traction de Boissier et Simon ;
- sa force musculaire, mesurée par l'épreuve des poids.

Les essais ont été pratiqués sur des groupes de 10 souris dont les performances motrices ont été mesurées à deux reprises : avant et une heure après

traitement par le SCS 289 administré, par voie digestive à des doses allant de 10 à 200 mg/kg, actives vis-à-vis des convulsions pentétrazoliques et des crises psychomotrices. A noter que l'épreuve de la planche à trous utilisée pour chiffrer la curiosité des animaux (décompte du nombre de visites des trous) n'est pratiquée qu'une seule fois, le comportement explorateur des animaux traités étant alors comparé à celui des témoins.

Les résultats obtenus dans chaque groupe ont permis de calculer la $DA_{50}$ capable de diminuer de 50 % les performances motrices initiales ou de diminuer de 50% la curiosité des souris traitées par rapport à celle des témoins.

INFLUENCE DE DOSES CROISSANTES DE SCS 289
SUR LA CURIOSITE DE LA SOURIS

| Doses de SCS 289 (mg/kg) | Nb de visites des trous* Traités | Témoins | Modifications de la curiosité |
|---|---|---|---|
| 10 | 27 | 26,5 | + 1,5 % |
| 25 | 27,4 | 26,4 | + 3,8 % |
| 50 | 23,6 | 24,2 | - 2,5 % |
| 75 | 21,4 | 27 | - 20,7 % |
| 100 | 8,6 | 25,4 | - 66,1 % |
| 150 | 0 | 24,2 | - 100,0 % |
| 200 | 0 | 24,2 | - 100,0 % |

* par souris pendant les cinq minutes d'observation

L'administration de SCS 289 affecte le comportement exploratoire de la souris et ce, d'autant plus que les doses mises en oeuvre sont plus élevées : la souris explore un moins grand nombre de trous lorsqu'on l'expose sur la planche à trous, indice d'une diminution de sa curiosité naturelle.

La diminution de la curiosité de la souris n'est manifeste qu'après administration de 75 mg/kg.

La curiosité est abolie à partir de 150 mg/kg.

La $DA_{50}$ capable de diminuer de 50 % la curiosité de la souris a été calculée par la méthode de Miller et Tainter ; elle est égale à : 82,5 ± 7,6 mg/kg.

---

INFLUENCE DE DOSES CROISSANTES DE SCS 289 SUR LES PERFORMANCES MOTRICES DE LA SOURIS

| Doses de SCS 289 mg/kg | Propriétés déficitaires motrices* sur l'agilité | la force musculaire | la motricité |
|---|---|---|---|
| 10 | 6,7 | 3,8 | 0 |
| 25 | 20 | 8,3 | 20 |
| 50 | 41,7 | 33 | 25 |
| 75 | 77,3 | 31,9 | 30 |
| 100 | 90 | 80,4 | 50 |
| 150 | 100 | 100 | 100 |
| 200 | 100 | 100 | 100 |

* Pourcentage de diminution des scores sous l'effet du traitement

---

L'administration de SCS 289 affecte la motricité spontanée, l'agilité et la force musculaire de la souris mais à des degrés divers selon les doses mises en oeuvre :

- la motricité n'est diminuée qu'à partir de la dose de 25 mg/kg et de façon modérée jusqu'à 75mg/kg. La $DA_{50}$ est égale à 120 mg/kg ;

- la force musculaire n'est abaissée réellement qu'après administration de doses de l'ordre de 25-50 mg/kg ; la $DA_{50}$ est égale à 63 mg/kg ;

- l'agilité est affectée dès la première dose expérimentée (10 mg/kg) et la $DA_{50}$ se situe à 42±7,7 mg/kg.

Toutes les performances sont abolies à 100 % sous l'effet de 150 ou 200 mg/kg de SCS 289.

Le comportement moteur spontané n'est altéré de façon notable qu'à des doses ($DA_{50}$ = 120 mg/kg) très supérieures aux doses anticonvulsivantes sur le pentétrazol et les crises psychomotrices.

Le traitement par le SCS 289 perturbe l'accomplissement des épreuves mettant en jeu la force musculaire et l'agilité des animaux à des doses inférieures à celles qui altèrent la motricité spontanée ($DA_{50}$ respectivement égales à 63 et 42 mg/kg).

La curiosité est émoussée par le SCS 289 à des doses intermédiaires ($DA_{50}$ de l'ordre de 82 mg/kg) 15 à 18 fois supérieures aux doses anticonvulsivantes selon que l'on envisage la protection contre les crises chimiques produites par le pentétrazol ou les crises psychomotrices produites par la stimulation électrique du cerveau à basse fréquence.

La SCS 289 peut affecter le comportement psychomoteur de la souris mais à des doses bien supérieures à celles qu'il faut utiliser pour protéger contre les crises pentétrazoliques ou électriques à basse fréquence.

La comparaison des index thérapeutiques absolus ($DL_{50}/DE_{50}$) et relatifs ($DE_{50}$ psychodépressive / $DE_{50}$ anticonvulsivante) du SCS 289 et du valproate de sodium donne les valeurs suivantes :

| $DL_{50}/DE_{50}$ PRODUITS | /pentétrazol•• | / Crises psychomotrices |
|---|---|---|
| SCS 289 | 153 | 511 |
| Valproate Na | 6,9 | 15 |
| $DE_{50}$ psychodépressive• / $DE_{50}$ anticonvulsivante | | |
| SCS 289 | + 5 | 16 |
| Valproate Na | + 3 | 7 |

• Valeur moyenne des $DE_{50}$ diminuant l'agilité, la force musculaire, la motricité et la curiosité

•• $DE_{50}$ déterminée sur SCS 289 = 15 mg/kg

Dans l'ensemble, le SCS 289 est plus maniable que le valproate de sodium ; selon que l'on considère la maniabilité absolue ou relative, c'est le SCS 289 qui s'avère le plus prometteur à l'égard des crises pentétrazoliques ou psychomotrices.

<u>Synergie avec le SCS 35</u>

Lors des recherches expérimentales, on a obsrvé d'intéressantes propriétés anticonvulsivantes pour les SCS 35 et SCS 289, le premier s'avérant un peu moins actif pour antagoniser les crises pentétrazoliques mais également moins psychodépresseur et, par ailleurs, non dépourvu d'action à l'égard des crises épileptiformes consécutives à l'électrochoc du lapin et de la souris. Aussi a-t-il paru intéressant de rechercher si l'association de ces deux aryl allophanates ne pouvait donner lieu à une synergie d'action dans le domaine anticonvulsivant.

Pour conduire à bien cette étude, on a parfois été mené à préciser l'action de l'un ou l'autre des deux produits SCS dans un domaine particulier.

Protection du lapin contre les crises épileptiformes consécutives à l'électrochoc

Ne considérant comme significative, que la protection complète, les traitements pratiqués une heure avant le second électrochoc ont conféré les protection suivantes (sur groupes de dix lapins) :

| Traitement | % d'animaux protégés complètement |
|---|---|
| SCS 35 100 mg/kg | Nul |
| SCS 35 200 mg/kg | 10 |
| SCS 289 100 mg/kg | 0 |
| SCS 289 200 mg/kg | 20 |
| SCS 35 100 mg/kg et SCS 289 100 mg/kg | 20 |
| SCS 35 100 mg/kg et SCS 289 200 mg/kg | 30 |
| SCS 35 200 mg/kg et SCS 289 100 mg/kg | 0 |
| SCS 35 200 mg/kg et SCS 289 200 mg/kg | 0 |

Aucun des produits SCS ne protège de façon complète lorsqu'ils sont administrés à la dose de 100mg/kg ; en association, ils protègent complètement 20% des animaux.

- Si, par ailleurs, on considère la protection contre les crises toniques (hyperextension des pattes postérieures), il apparait également une synergie particulièrement aux doses associées de 200 mg/kg de produits.

| Traitement | % d'animaux protégés contre les seules crises toniques |
|---|---|
| SCS 35 100 mg/kg | 60 |
| SCS 35 200 mg/kg | 80 |
| SCS 289 100 mg/kg | 60 |
| SCS 280 200 mg/kg | 50 |
| SCS 35 100 mg/kg et SCS 289 100 mg/kg | 40 |
| SCS 35 100 mg/kg et SCS 289 200 mg/kg | 70 |
| SCS 35 200 mg/kg et SCS 289 100 mg/kg | 100 |
| SCS 35 200 mg/kg et SCS 289 200 mg/kg | 100 |

Chacun des produits SCS expérimentés SCS 35 et SCS 289 n'exerce qu'une action très incomplète et relativement modeste vis-à-vis des crises épileptiformes déclenchées par électrochoc chez le lapin ; il apparait que leur association permet d'améliorer la protection.

<u>Protection contre les crises psychomotrices de la souris</u>

Vis-à-vis des crises psychomotrices provoquées chez la souris par une stimulation électrique transcranienne à basse fréquence, le SCS 35 apparait plus actif que le SCS 289 :

| Traitement | Pourcentage de protection |
|---|---|
| SCS 35 2,5 mg/kg | 40 |
| SCS 35 5 mg/kg | 60 $DE_{50}$ = 3,5 mg/kg |
| SCS 35 10 mg/kg | 90 |

la dose de 2,5 mg/kg de SCS 289 protégeant également 40% des animaux mais la $DE_{50}$ se situant aux environs de 5,5 mg/kg.

Si l'on traite les animaux par un mélange de 2,5 mg/kg de chacun des deux SCS expérimentés, la protection n'est que de 45 %.

Il n'est pas constaté de synergie d'action entre les SCS 35 et les SCS 289 à l'égard des crises psychomotrices de la souris.

<u>Protection contre les crises pentétrazoliques de la souris</u>

Pour déceler une synergie d'action antagoniste vis-à-vis des effets convulsivants et létaux du pentétrazol injecté par voie sous-cutanée chez la souris à la dose de 125 mg/kg, on a déterminé avec plus de précision la $DE_{50}$ du SCS 289 et on a recherché si l'association avec le SCS 35 apportait un surcroît appréciable d'activité.

On n'a retenu comme significative que la protection à l'égard des effets létaux observés sur 24 heures.

| Traitement préalable 1 h avant pentétrazol | % de létalité en 24 h* |
|---|---|
| Sans | 100 |
| SCS 289 10 mg/kg | 80 |
| SCS 289 20 mg/kg | 30 |
| SCS 289 30 mg/kg | 0 |
| SCS 289 40 mg/kg | 0 |
| SCS 289 50 mg/kg | 0 |
| SCS 289 10 mg/kg et SCS 35 10 mg/kg | 30 |
| SCS 289 20 mg/kg et SCS 35 10 mg/kg | 15 |
| SCS 289 30 mg/kg et SCS 35 10 mg/kg | 0 |
| SCS 289 40 mg/kg et SCS 35 10 mg/kg | 0 |
| SCS 289 50 mg/kg et SCS 35 10 mg/kg | 0 |
| SCS 289 10 mg/kg et SCS 35 25 mg/kg | 25 |
| SCS 289 20 mg/kg et SCS 35 25 mg/kg | 15 |
| SCS 289 30 mg/kg et SCS 35 25 mg/kg | 0 |
| SCS 289 40 mg/kg et SCS 35 25 mg/kg | 0 |
| SCS 289 50 mg/kg et SCS 35 25 mg/kg | 0 |
| SCS 289 10 mg/kg et SCS 35 50 mg/kg | 0 |
| SCS 289 10 mg/kg et SCS 35 100 mg/kg | 0 |
| SCS 289 25 mg/kg et SCS 35 25 mg/kg | 0 |

* sur groupes individuels de 20 souris par dose expérimentée

Il apparaît donc que l'action protectrice du SCS 289 à l'égard des effets létaux du pentétrazol sont très nettement majorés lorsqu'on lui associe du SCS 35 à une dose inactive (10 mg/kg) ou à peine active, (25mg/kg).

0187057



TAUX DE PROTECTION EN POURCENTAGE
VIS-A-VIS DE LA LETALITE PENTETRAZOLIQUE

| Traitement préalable | % de protection |
|---|---|
| SCS 289 10 mg/kg | 20 |
| SCS 289 20 mg/kg | 70 |
| SCS 289 30 mg/kg | 100 |
| SCS 289 40 mg/kg | 100 |
| SCS 289 50 mg/kg | 100 |
| | |
| SCS 35 10 mg/kg | 0 |
| SCS 25 20 mg/kg | 10 |
| SCS 35 50 mg/kg | 50 |
| | |
| SCS 289 10 mg/kg et SCS 35 10 mg/kg | 70 |
| SCS 289 20 mg/kg et SCS 35 10 mg/kg | 85 |
| SCS 289 30 mg/kg et SCS 35 10 mg/kg | 100 |
| SCS 289 40 mg/kg et SCS 35 10 mg/kg | 100 |
| | |
| SCS 289 10 mg/kg et SCS 35 25 mg/kg | 75 |
| SCS 289 20 mg/kg et SCS 35 25 mg/kg | 85 |
| SCS 289 30 mg/kg et SCS 35 25 mg/kg | 100 |
| SCS 289 40 mg/kg et SCS 35 25 mg/kg | 100 |
| | |
| SCS 289 10 mg/kg et SCS 35 50 mg/kg | 100 |
| SCS 289 10 mg/kg et SCS 35 100 mg/kg | 100 |
| | |
| SCS 289 25 mg/kg et SCS 35 25 mg/kg | 100 |
| SCS 289 25 mg/kg et SCS 35 50 mg/kg | 100 |

En effet, l'administration de 10 mg/kg de SCS 289 est suivie d'une protection très notable (75 à 100 % au lieu de 20 %) si on l'additionne de SCS 35 à des doses inactives (10 mg/kg) ou développant des effets protecteurs de l'ordre de 10 à 50 % (25 ou 50 mg/kg).

Il est donc noté une très nette synergie dans les effets protecteurs qu'exercent les SCS 35 et SCS 289 à l'égard de la létalité pentétrazolique chez la souris.

<u>Action sur le comportement psychomoteur de la souris</u>

L'association des SCS 289 et SCS 35 ne peut être bénéfique que dans la mesure où les effets anticonvulsivants sont majorés sans toutefois que le comportement psychomoteur soit affecté. Aussi a-t-on recherché si l'association de doses faibles entraînant une augmentation des effets protecteurs à l'égard des crises pentétrazoliques apportait une modification notable du comportement des souris.

Les expérimentations comportementales précédemment décrites ont été appliquées à des animaux recevant SCS 35 et SCS 289 aux doses de 10 et 25 mg/kg. Les résultats obtenus ont été les suivants :

| Traitement (en mg/kg) | Effets psychodépresseurs | | | |
| --- | --- | --- | --- | --- |
| | Agilité | force musculaire | curiosité | motilité |
| SCS35 (10)&SCS289 (10) | 6,7 | 2,5 | 6,1 | 0 |
| SCS35 (25)&SCS289 (10) | 23,3 | 7,8 | 16 | 0 |
| SCS35 (25)&SCS289 (25) | 33,3 | 14,3 | 29,4 | 40 |
| Rappel SCS 289 (10) | 6,7 | 3,8 | (+ 1,5) | 0 |
| SCS 289 (25) | 20 | 8,3 | (+ 3,8) | 20 |

Qu'il soit administré seul ou associé au SCS 35, à la dose de 10 mg/kg, le SCS 289 n'altère pas de façon significative le comportement psychomoteur de la souris. Lorsqu'à la dose de 10 mg/kg de SCS 289, on associe 25 mg/kg de SCS 35, seules l'agilité et la curiosité sont affectées. Ce n'est que l'association des

deux produits à la dose de 25 mg/kg de chacun d'entre eux qui entraîne une véritable dépression.

Or, il semble que vis-à-vis des crises pentétrazoliques, on puisse tirer profit d'une association avec des doses inférieures.

Il est donc possible d'envisager une synergie d'effets anticonvulsivants sans avoir à redouter une potentialisation des effets psychodépresseurs.

En conclusion, il résulte de ce qui précède que l'aryl allophanate d'isopropyle SCS 289 est très peu toxique par voie digestive :

- chez la souris : DMT = 1500 mg/kg, $DL_{50}$ = 2,30 g/kg
- chez le rat : DMT = 500 mg/kg $DL_{50}$ = 2,0 g/kg.

L'existence de propriétés anticonvulsivantes a été confirmée :

- vis-à-vis des myoclonies palpébrales du lapin, le produit s'avérant deux fois plus actif que la phénacétamide et dix fois plus actif que le valproate de sodium ;

- à l'égard des crises pentétrazoliques maximales et létales du pentétrazol chez la souris, le composé expérimenté possédant une $DE_{50}$ chiffrée à 15 mg/kg (alors que la $DE_{50}$ du SCS 35 était égale à 55 mg/kg) ;

- contre les crises psychomotrices produites chez la souris par une stimulation électrique du cerveau à basse fréquence, le SCS 289 s'avérant un peu moins actif que le SCS 35 ($DE_{50}$ égale à 4,5 mg/kg au lieu de 3,5) mais trois fois plus actif que la phénacétamide et 26 fois plus actif que le valproate de sodium.

Il a été vérifié, chez le lapin, que l'activité est insignifiante à l'égard des crises électriques épileptiformes consécutives à l'électrochoc.

L'administration de doses croissantes a permis de préciser l'importance des effets dépresseurs sur le comportement psychomoteur de la souris ; selon que l'on envisage la dépression de l'agilité, de la force musculaire, de la curiosité ou de la motilité spontanée, la $DA_{50}$ passe de 42 à 63-82,5 et 120 mg/kg.

Considérant la maniabilité du produit ($DL_{50}/DE_{50}$) et la comparant à celle du SCS 35 et du valproate de sodium expérimentés dans des conditions analogues, il apparaît que vis-à-vis des crises pentétrazoliques de la souris, SCS 289 est bien supérieur au valproate de sodium.

L'établissement d'un index thérapeutique, dose dépressive 50 % sur le SNC/$DE_{50}$ anticonvulsivante, place toujours les SCS avant le valproate de sodium mais selon que l'on envisage la protection contre les crises psychomotrices ou contre les crises pentétrazoliques maximales de la souris, c'est le SCS 289 qui apparaît le plus maniable.

Ayant recherché si l'association des deux produits SCS 289 et SCS 35 pouvait être bénéfique, on a constaté que, dans certaines conditions (de doses), les effets anticonvulsivants à l'égard de l'électrochoc du lapin et des crises pentétrazoliques maximales de la souris se trouvaient majorés alors que les effets dépresseurs sur le système nerveux central ne donnaient lieu à une synergie qu'à partir de certaines doses. Autrement dit, il est possible d'envisager une synergie d'action anticonvulsivante entre SCS 35 et SCS 289 en mettant en oeuvre des doses qui ne dépriment pas le système nerveux central : à titre d'exemple, l'association de 10 mg/kg de SCS 35 et 10 mg/kg de SCS 289 ne modifie pratiquement pas le comportement psychomoteur de la souris et, cependant, assure une protection de l'ordre de 70 % des animaux traités vis-à-vis des crises pentétrazoliques maximales.

REVENDICATIONS

1. A titre de produit industriel nouveau, le phényl-2 allophanate d'isopropyle répondant à la formule:

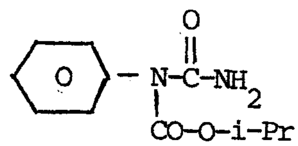

dans laquelle :
i-Pr désigne le radical isopropyle.

2. Procédé de fabrication du phényl-2 allophanate d'isopropyle caractérisé par le fait qu'il consiste à hydrolyser le phényl isopropyloxycarbonyl cyanamide.

3. Procédé selon la revendication 2, caractérisé par le fait que cette hydrolyse est effectuée en milieu acide en présence ou non d'un solvant organique miscible à l'eau et en opérant à chaud.

4. Procédé selon la revendication 3, caractérisé par le fait que le milieu acide peut être constitué par une solution d'acide chlorhydrique ; le solvant organique s'il est présent peut être l'éthanol ou l'acide acétique et la température de la réaction est avantageusement la température de reflux du mélange réactionnel.

5. Procédé selon l'une quelconque des revendications 2 à 4, caractérisé par le fait que le phénylisopropyloxycarbonyl cyanamide appliqué comme composé de départ est lui-même un produit nouveau pouvant résulter de l'acylation du phényl cyanamide par le chloroformiate d'isopropyle.

6. Procédé selon la revendication 5, caractérisé par le fait que l'on prépare avantageusement et intermédiairement un dérivé métallique du type sel de sodium ou de potassium du phényl cyanamide avant réaction avec le chloroformiate d'isopropyle.

7. Procédé selon la revendication 6, caractérisé par le fait que ledit dérivé métallique est simplement obtenu en opérant dans un milieu aqueux et en dissolvant le phényl cyanamide dans une solution aqueuse contenant le quantité équimoléculaire de soude ou de potasse.

8. Procédé selon la revendication 6, caractérisé par le fait que l'on additionne au phényl cyanamide une solution aqueuse de soude ou de potasse et le chloroformiate d'isopropyle par une réaction de Schotten-Baumann.

9. Procédé selon la revendication 6, caractérisé par le fait qu'on réalise la réaction par transfert de phase.

10. Application du phényl-2 allophanate d'isopropyle selon la revendication 1 dans le domaine pharmaceutique, en particulier dans le domaine où l'on recherche particulièrement les propriétés anticonvulsivantes marquées vis-à-vis des crises psychomotrices et des crises pentétrazoliques.

11. Médicament à base de phényl-2 allophanate d'isopropyle selon la revendication 1 en vue de l'application selon la revendication 10.

12. Médicament selon la revendication 11 contenant le phényl-2 allophanate d'isopropyle selon la revendication 1 en association avec du phényl-2 allophanate d'éthyle.

Office européen des brevets

# RAPPORT DE RECHERCHE EUROPEENNE

Numéro de la demande

EP 85 40 2147

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | FR-A-2 271 202 (SUCRERIES DU SOISSONNAIS ET COMPAGNIE SUCRIERE)<br>* Revendications *<br>--- | 1-9 | C 07 C 127/22<br>A 61 K 31/27 |
| A | FR-A-2 409 752 (SUCRERIES DU SOISSONNAIS ET COMPAGNIE SUCRIERE)<br>* Revendications *<br>----- | 10-12 | |
| | | | DOMAINES TECHNIQUES RECHERCHES (Int. Cl.4) |
| | | | C 07 C 127/00 |

Le présent rapport de recherche a été établi pour toutes les revendications

| Lieu de la recherche | Date d'achèvement de la recherche | Examinateur |
|---|---|---|
| LA HAYE | 13-02-1986 | GAUTIER R.H.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82